# EUROPEAN PATENT SPECIFICATION

(11) **EP 1 638 535 B1**
(45) Date of publication and mention of the grant of the patent: **25.07.2007**
(21) Application number: 04741886.8
(22) Date of filing: 24.06.2004
(51) Int. Cl.: A61K 9/22

(54) **SUBCUTANEOUS IMPLANTS HAVING LIMITED INITIAL RELEASE OF THE ACTIVE PRINCIPLE AND SUBSEQUENT LINEARLY VARYING EXTENDED RELEASE THEREOF**
SUBCUTANE IMPLANTATE MIT BEGRENZTER INITIALER WIRKSTOFF-FREISETZUNG UND DEREN ANSCHLIESSENDE LINEARE VERÄNDERLICHE VERLÄNGERTE FREISETZUNG
IMPLANTS SOUS-CUTANES A LIBERATION INITIALE DE PRINCIPE ACTIF CONTROLEE, PUIS A LIBERATION PROLONGEE A VARIATION LINEAIRE

(30) Priority: 26.06.2003 IT MI20031299; 26.06.2003 IT MI20031301; 26.06.2003 IT MI20031300; 26.06.2003 IT MI20031303
(43) Date of publication of application: 29.03.2006
(73) Proprietor: Mediolanum Pharmaceuticals Limited, Dublin 4 (IE)
(72) Inventor: MAURIAC, Patrice, F-75012 Paris (FR); MARION, Pierre, F-93360 Neuilly Plaisance (FR)
(74) Representative: Gervasi, Gemma
(86) International application number: PCT/EP2004/051230
(87) International publication number: WO 2005/000278

(56) References cited:
- WO-A-00/33809
- WO-A-03/051328
- WO-A-03/094888
- PHARMACEUTICAL RESEARCH, vol. 13, no. 7, 1996, pages 1059-1064, XP008036235
- NEGRIN C M ET AL: "Methadone implants for methadone maintenance treatment. In vitro and in vivo animal studies" JOURNAL OF CONTROLLED RELEASE, ELSEVIER SCIENCE PUBLISHERS B.V. AMSTERDAM, NL, vol. 95, no. 3, 24 March 2004 (2004-03-24), pages 413-421, XP004496381 ISSN: 0168-3659

## Description

### FIELD OF THE INVENTION

The present invention relates to subcutaneous implants having limited initial release of the active principle and subsequent linearly varying extended release thereof.

### STATE OF THE ART

The advantage of using implants containing controlled release drugs is well known in the state of the art. Many therapeutic agents are rapidly metabolized and eliminated by the human or mammalian organism, therefore requiring frequent administration of the drug with the aim of maintaining an adequate therapeutic concentration.

Some controlled release implants are of the "matrix" type. In other words, an active principle is dispersed in the matrix consisting of a polymeric material of porous or non-porous type, which is solid or semi-solid, and permeable or impermeable to the active principle.

The matrix devices can be biodegradable i.e. can slowly erode, or they can be non-degradable; in this case the active principle diffuses across the wall or pores of the matrix.

An example of controlled release implants are represented by subcutaneous implants.

A particular use of such implants is for the administration of peptides.

For example USP 4,767,628 describes compositions containing a peptide and a polymer based on lactic acid, or a lactic acid-glycolic acid copolymer.

These compositions are prepared by the following method. The peptide and the (co)polymer are dissolved in a solvent which can be the same or different for both the said substances, then the two solutions are mixed. The solvent is subsequently removed at low temperature and the powder thus obtained is extruded.

The compositions contemplated in this patent can also be used for preparing subcutaneous implants, as stated in the subsequent patent, US 5,366,734.

The release mechanism in these types of implants takes place in the following manner. The lactic acid-glycolic acid copolymer is incompatible with the peptide therefore the diffusion of the active principle through the polymer is incompatible.

When these implants are introduced into a buffered aqueous solution at 37°C, the water penetrates and diffuses into the implant and becomes distributed between the polymer and the peptide, partially hydrating the peptide.

The first release stage of the peptide in such a type of implant, described in USP 5,366,734, is a diffusion stage caused by the swelling of the polymer.

With the swelling of the polymer, canaliculi of hydrated peptide are formed where the peptide diffuses outwards.

When the polymer ceases to swell, the active principle is no longer released.

The second release stage is caused by the degradation of the polymer. During this stage, holes and fractures in the matrix form to allow the release of hydrated peptide which is still within the matrix.

The maximum period of time for release obtained with these types of implants is about 3 months.

The fundamental characteristics of the compositions for subcutaneous implants described in the previous aforementioned patents reside in the fact that the particle density distribution of the peptide in the polymeric substance is homogeneous.

In WO98/09613 a process for preparing subcutaneous implants capable of releasing active principles consisting of peptides is described.

This process comprises the following stages:
- grinding a copolymer based on lactic acid-glycolic acid,
- treating the copolymer with an aqueous peptide slurry (in the examples the treatment of the copolymer with an aqueous solution of a peptide salt is described in place of the treatment with a polypeptide slurry), and relative mixing to obtain a homogenous mixture,
- drying the mixture obtained at a temperature not greater than 25°C,
- extruding the mixture at 70-110°C and obtaining small cylinders for use as subcutaneous implants.

The compositions for subcutaneous implants described in the aforestated prior patents are characterised in that the peptide presents a homogenous distribution density because solutions of the active principle are used.

Even commercially available subcutaneous implants have the disadvantage of releasing this type of active principle for a time not longer than 3 months.

The subcutaneous implants described in WOOO/33809 represent a net improvement with reference to previous subcutaneous implants containing as active principle a peptide dispersed in a matrix of polylactic-glycolic acid in that they are able to release the aforesaid active principle in 6 months.

These implants are different from those used previously in that the particles of peptide present extremely heterogeneous dimensions which vary between 1 micron and 63 microns.

These implants are prepared with a process that contemplates in particular the following stages:
- dry mixing the peptide in the form of particles with heterogeneous dimensions which vary within the aforesaid range, with powdered polylactic-glycolic acid (PLGA),
- wet granulating the mixture obtained from the preceding stage using a suitable solvent,
- drying the granulated product to obtain a residue containing a minimum liquid content of between 0.1 and 3%,
- extruding the mixture obtained from the previous stage,
- cutting the extruded product to the dimensions suitable for a subcutaneous implant.

The subcutaneous implants described in said previous patents differ also in that they present an essentially triphasic and not biphasic release profile as clarified in the following manner: release by pure diffusion, diffusion by swelling and release by polymer degradation.

This progression therefore allows for an extension of release times. In fact when these implants are introduced into an aqueous medium, the water diffuses through the polymeric matrix reaching the peptide particles closest to the surface and subsequently the more interior zones.

The implant remains substantially unmodified for about 6 weeks and in this period releases approximately 30% of the peptide.

The duration of this stage of pure diffusion is essentially determined by the level of heterogeneity of the peptide dimensions and the rate is essentially determined by the particle content in the PLGA matrix.

As the active principle presents a diversity of dimensions, a sufficient quantity of peptide remains after the first stage of dissolution and can be released in the successive stages mentioned, that is release by diffusion and swelling, or release by disintegration of the polymer.

All these types of aforestated subcutaneous implants, suffer from a drawback essentially caused by the fact that once the subcutaneous implants are administered in the human body, high total amounts of active principle can be attained (in some cases decidedly greater than maximum permitted daily dosages).

An immediate dissolution of the active principle can therefore occur; this phenomenon, which does not deplete in subsequent days but at times increases in a scalar progression, is known as initial "burst". In such cases, therefore, it can be verified that the quantity of drug released from such systems, even when compared to the quantity of total active principle contained in the subcutaneous implants administered may be low, can in some cases be considered dangerous if with such an initial burst the maximum permitted daily dosage for such a type of drug is approached or exceeded.

In addition, even if the aforesaid drawbacks are not present with some active principles and with some pathologies, it can be useful not to release the active principle immediately but to dose its release in a more gradual manner. The need was felt to provide a subcutaneous implant which complies with the aforesaid requirements:
- does not allow the immediate dissolution of the active principle at time t=0;
- releases the active principle through the core (i) and the coating (ii) by diffusion, and the resulting release rate by diffusion of the active principle is lower than that of an uncoated implant so as to reduce the initial burst which occurs in the first days after insertion of the implant;
- after release by pure diffusion, the remaining quantity of active principle and consequently release rate is higher in the second phase of active principle release,
- able to limit the second burst caused by the disintegration of the core (i).

In US 6,022,554 a coating is described for slow release implants consisting of an insoluble polymer among which is mentioned PLGA and in which the presence of polyethylene glycol is indispensable as an agent able to form pores in the insoluble polymer and therefore able to control active principle release. US 6,319,512 describes a coated subcutaneous implant wherein the coating comprises a polymeric film prepared prior to the formation of the core wherein such film comprises a mixture of polylactic acid with a molecular weight between 2000 and 6000 Da and a copolymer based on polylactic-glycolic acid with a molecular weight between 20,000 and 100,000 Da and with a lactic acid/glycolic acid molar ratio between 60:40 and 40:60.

### SUMMARY OF THE INVENTION

The Applicant has now unexpectedly found a subcutaneous implant which overcomes the drawbacks of subcutaneous implants of the state of the art, in which the active principle is dispersed in PLGA.

The present invention therefore provides subcutaneous implants comprising:
- a core (i) comprising at least one active principle dispersed in a polymeric matrix essentially consisting of polylactic- glycolic acid (PLGA), obtained by extrusion,
- a coating (ii) in film form comprising as the main component polylactic- glycolic acid(PLGA).

With the subcutaneous implants of the present invention, immediate drug dissolution can in fact be reduced as no active principle is available to be released. The rate of diffusion at the first stage of release is lower, hence initial burst release is reduced.

### DESCRIPTION OF THE FIGURES

Figure 1A shows an enlarged(150x) cross section image taken at the optical microscope Zeiss (Model Stemi 2000-C) of one of the coated subcutaneous implant of example 1.
Figure 1B shows a diagram of in-vitro release of the coated subcutaneous implant of the present invention, prepared as described in example 1 (and compared with release of the same uncoated subcutaneous implant), where the y-axis shows the total quantity of active principle released (mg) and the x-axis shows time in days.
Figure 2A shows an enlarged (300x) cross section photo made with the above optical microscope of one of the coated subcutaneous implant of example 2
Figure 2B shows a diagram of in-vitro release profile of the coated subcutaneous implant of the present invention, prepared as described in example 2 and compared with release of the same uncoated subcutaneous implant., where the y-axis and the x-axis have the aforementioned meanings.
Figure 3A shows an enlarged (150x) cross section image taken the above optical microscope of one of the coated subcutaneous implant of the invention of example 3.
Figure 3B shows a diagram of in-vitro release of the coated subcutaneous implant of the present invention, prepared as described in example 3 and compared with release of the uncoated same subcutaneous implant, where the y-axis and the x-axis have the aforementioned meanings.
Figure 4A shows the enlarged (75X) cross-section image taken at the above optical microscope of one of the coated subcutaneous implant prepared of Example 4 (coating thickness 140µm).
Figure 4B shows the in vitro release profile of the active principle from the coated subcutaneous implants of Example 4, where the y-axis and the x-axis have the aforementioned meanings.
Figure 5A shows the enlarged (75X) cross-section image taken at the above optical microscope of one of the coated subcutaneous implants of Example 5 (coating thickness 120 µm).
Figure 5B shows the in vitro release profile of the active principle from the coated subcutaneous implants of Example 5, where the y-axis and the x-axis have the aforementioned meanings.
Figure 5C shows the in vitro release profile of the active principle from the coated subcutaneous implants of Example 5 compared with the release profile obtained with those of Example 4, where the y-axis and the x-axis have the aforementioned meanings.
Figure 6A shows the enlarged (75X) cross-section photo made at the above optical microscope of one of the subcutaneous coated implants of Example 6 (coating thickness 80 µm).
Figure 6B shows the in vitro release profile of the active principle from the cylindrical coated subcutaneous implants of Example 6, where the y-axis and the x-axis have the aforementioned meanings.
Figure 6C shows the in vitro release profile of the active principle from the cylindrical subcutaneous implants of Example 6 compared with the corresponding release profile obtained with those of Example 4, where the y-axis and the x-axis have the aforementioned meanings.
Figure 7A shows an enlarged (75X) cross-section image taken at the above optical microscope of one of the coated subcutaneous implants of Example 7 (coating thickness 200 µm).
Figure 7B shows the in vitro release profile of the active principle from the cylindrical coated subcutaneous implants of example 7, where the y-axis and the x-axis have the aforementioned meanings.
Figure 7C shows the in vitro release profile of the active principle from the coated subcutaneous implants of Example 7, compared with the corresponding release profile obtained with those Example 4, where the y-axis and the x-axis have the aforementioned meanings.
Figure 7D shows the in vitro release profile in the first 14 days of the coated subcutaneous implants disclosed in Example 4 and in Example 7 (coating thickness 120µm)
Figure 8A shows the enlarged (75X) cross-section image taken at the above optical microscope of one of the coated subcutaneous implants of Example 8 (coating thickness 50 µm).
Figure 8B shows the in vitro release profile of the active principle from the coated subcutaneous implants compared with the uncoated subcutaneous implants of Example 8, where the y-axis and the x-axis have the aforementioned meanings.
Figure 9A shows the enlarged (75X) cross-section photo made at the above optical microscope of one of the coated subcutaneous implants of Example 10 (coating thickness 100 µm).
Figure 9B shows the in vitro release profile of the active principle from the coated subcutaneous implants of Example 10, where the y-axis and the x-axis have the aforementioned meanings.
Figure 10A shows an enlarged (75X) cross-section image taken at the above optical microscope of one of the coated subcutaneous implants of Example 11 (coating thickness 150 µm).
Figure 10B shows the in vitro release profile of the active principle from the coated subcutaneous implants of Example 11, where the y-axis and the x-axis have the aforementioned meanings.
Figure 11 shows the in vitro release profile of the active principle from the coated subcutaneous implants of Example 13 compared with respectively those obtained with the subcutaneous implants of examples 12 and 10 (coating thickness 150 µm), where the y-axis and the x-axis have the aforementioned meanings.
Figure 12A shows the enlarged (75X) cross-section photo made at the above optical microscope of one of the coated subcutaneous implants of Example 15.
Figure 12B shows the in vitro release profile of the active principle from the subcutaneous implants compared with that obtained with the subcutaneous implants of Example 14, where the y-axis and the x-axis have the aforementioned meanings.
Figure 13 shows the in vitro release profile of the active principle from the coated subcutaneous implants of Example 16 compared with that obtained with the subcutaneous implants of Example 15.
Figure 14 shows a schematic view in section of the cylindrical co-extruder used for preparing the subcutaneous implants of the present invention.

### DETAILED DESCRIPTION OF THE INVENTION

The coated subcutaneous implants of the present invention preferably have a core containing the active principles chosen from peptides, active principles able to increase bone density, analgesic-narcotic active principles, active principles consisting of steroid hormones for hormonal treatments during menopause and for contraception.

Preferably the core (i) of the coated implants, containing a peptide, corresponds to the subcutaneous implants disclosed in WO00/33809, and more preferably said peptides are chosen from: avorelin, triptorelin, goserelin, leuprorelin.

The coated subcutaneous implants whose core contain other active principles dispersed in a PLGA matrix are for example the following:
A) a core containing at least one active principle able to increase bone density in association with PLGA.
   The active principle present in the core (A) of the coated subcutaneous implants can present heterogeneous dimensions or can have a more homogeneous particle size.
B) core containing an analgesic-narcotic active principle in association with polylactic-glycolic acid (PLGA).
C) a core containing a steroid hormone, for hormone treatments during menopause and for contraception, dispersed in a matrix essentially consisting of polylactic-glycolic acid (PLGA).

The aforementioned cores (A), (B) and (C) can be prepared by a process which comprises the following stages:
I) dry mixing the active principle ,
II) possibly granulating the mixture obtained from stage (I) and drying the granules thus obtained,
III) extruding the mixture obtained from (I) or from (II) and cutting the extruded product to obtain small cylinders of dimensions suitable for obtaining subcutaneous implants.

The active principles contained in the core (A) able to increase bone density are preferably chosen from: pharmaceutically acceptable bisphosphonic acids and their salts, vitamin D or analogues thereof and sex hormones.

Of these bisphosphonic acids and their pharmaceutically acceptable related salts of general formula (I): 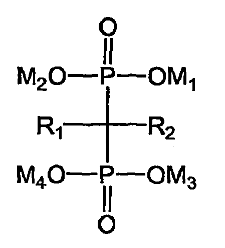 in which M₁, M₂, M₃ and M₄ are monovalent cations and/or H, where said monovalent cations are chosen from alkaline metals, or cations of aliphatic or cycloaliphatic amines, and even more preferably said cations are Na⁺, we would cite for example those in which R₁ and R₂ have the meanings given in the following table 1:

**Table 1**

| Bisphosphonate | R₁ | R₂ |
|---|---|---|
| Etidronate | OH | CH₃ |
| Chlodronate | Cl | Cl |
| Pamidronate | OH | CH₂CH₂NH₂ |
| Alendronate | OH | CH₂CH₂CH₂NH₂ |
| Risedronate | OH | CH₂-3-pyridine |
| Tiludronate | H | CH₂-S-phenyl-4Cl |
| Ibandronate | OH | CH₂CH₂N(CH₃)pentyl |
| Zoledronate | OH | CH₂CH₂-1-imidazole |
| Minodronate | OH | CH₂CH₂-2-imidazopyridinyl |
| Incadronate | OH | N-(cycloheptyl) |
| Olpadronate | OH | CH₂CH₂N(CH₃)₂ |
| Neridronate | OH | CH₂CH₂CH₂CH₂CH₂NH₂ |
| EB1053 | OH | CH₂-1-pyrrolidinyl |

Particularly preferred are the cores (A) containing etidronate disodium, alendronate disodium and pamidronate disodium.

Preferably the core (A) contains preferably calcitriol as the analogue of vitamin D.

The "sex hormones" used both in the cores (A) are chosen from the class consisting of estrogens and progestins, and of the latter, androgenic progestins are preferably used.

Preferably the cores (A) of the present invention contain estrogens of steroid type chosen from the class consisting of estradiol, estradiol valerate, estradiol cypionate, estrone, estrone sulphate or estrogens of non-steroidal type for example diethylstilbestrol, p-p'-DDT, bis-phenyl-A.

The same cores (A) or (C) preferably contain male progestins chosen from the class consisting of norethindrone, norethinodrel, norgestrel, desogestrel, norgestimate.

The "drugs with narcotic analgesic activity", contained in the core (B) are preferably morphine and morphinans, i.e. compounds having a chemical structure and activity similar to that of morphine i.e. µ receptor agonists, but also compounds with morphinic-type activity, in other words also µ receptor agonists but with a different chemical structure such as those belonging to the phenylpiperidine class. (Goodman & Gilman's "The pharmacological basis of therapeutics "Ninth Edition Chapter 23 pages 521-555).

Within the class of phenylpiperidine µ receptor agonists, the core (B) of the coated subcutaneous implants according to the present invention contain preferably at least one active principle chosen from the class consisting of meperidine, fentanyl and relative pharmaceutically acceptable salts fentanyl congeners, for example sufentanyl, alfentanyl, lofentanyl, carfentanyl, remifentanyl and their pharmaceutically acceptable salts.

According to a particularly preferred embodiment the core of the present invention contain in particular fentanil citrate as active principle.

The steroid hormones contained in the core (C) of the subcutaneous implants according to the present invention are preferably the aforementioned estrogens of steroid type and progestins used for the treatment of menopause and for contraception.

The core (C) of the coated subcutaneous implants preferably contain as the active ingredient merdoxyprogesterone acetate.

Preferably the subcutaneous implants of the present invention can have the core (i) prepared as described in US 4,767,628, US 5,366,374, WO98/09613, WO00/33809, or with the aforementioned process used in preparing the cores (A), (B) or (C).

The PGLA used in the core (i) presents preferably a molecular weight of between 50,000 and 150,000 and a molar ratio of the lactic acid to glycolic acid monomers between 50:50 and 95:5.

With the wording relating to the core (i) "essentially consisting of", the Applicant means that the PLGA in the polymeric matrix is present in amounts higher or equal to 99,9%.

With the wording relating to the coating "comprising as the main component polylactic-glycolic acid (PLGA)" the Applicant means that the polylactic-glycolic acid is contained in the coating in amounts ranging from 60 to 100%, more preferably in amounts from 75 to 99.999 %, wherein the remaining to 100% essentially consists of excipients and/or the same active principle used in the core (i).

According to a preferred embodiment the coating (ii) essentially consists of polylactic-glycolic acid, namely the PLGA is present in amounts equal or higher than 99,9%.

According to another preferred embodiment the coating consists of a mixture of PLGA in amounts of 80% and at least one hydrophilic excipient preferably polyvinyl pyrrolidone, D-mannitol or mixtures thereof in amounts of 20%

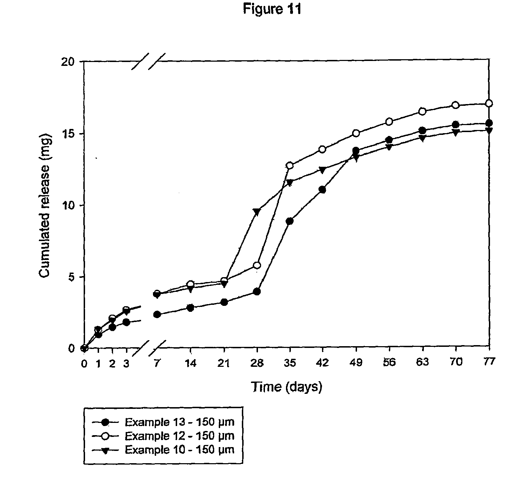

If compared to the coating containing as the sole component polylactic-glycolic acid, this latter type of coating allows to obtain a fairly constant release rate for a more protracted period of time (see figure 11).

According to another preferred embodiment the coating (ii) consists of a mixture of PLGA in amounts of 75% and the active ingredient used in the core (i) in amounts of 25%.

If compared to the coating containing the sole PLGA, the latter allows a higher amounts of active ingredient (see Figure 7C), in addition with the latter coating it is possible to have a much more linear relase pattern (see Figure 7D)

The polylactic-glycolic acid (PLGA) present in the coating (ii) has an average molecular weight preferably between 50,000 and 150,000 and a molar ratio of the lactic acid to glycolic acid monomers preferably between 50:50 and 95:5.

Even more preferably the molecular weight is between 100,000 and 150,000 and the molar ratio of lactic acid-glycolic acid monomers is between 50/50 and 75/25.

The subcutaneous implants according to the present invention can be prepared with a process that comprises the following stages:
a) preparing the core (i) containing the active principle, by extrusion,
b) passing the core (i) into a PLGA solution in a suitable solvent preferably chosen from: apolar solvents, preferably chlorinated solvents, even more preferably methylene chloride, aprotic polar solvents preferably chosen from: acetonitrile, ethyl acetate, tetrahydrofuran so that said cores remain in contact with said solution for a contact time of between 1 and 5 seconds, preferably 1 second,
c) drying the aforesaid cores obtained from stage (b).

Preferably the concentration of the PLGA solution in the solvent used in stage (a) is between 70 and 300 g/l and even more preferably between 100 and 200 g/l.

The subcutaneous implants of the present invention can be prepared using a process consisting of co-extruding the mixture of active principle and PLGA forming the core (i) with the coating in film form.

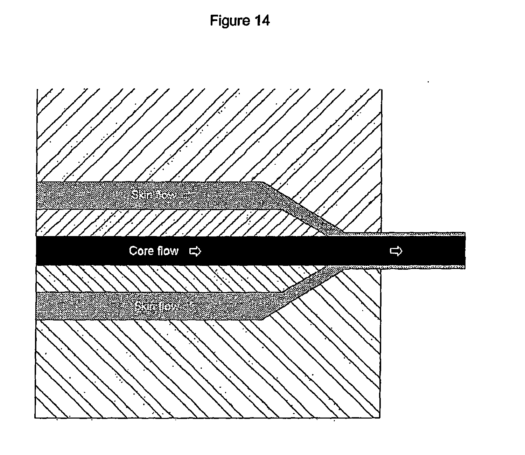

Typically the term co-extrusion means the simultaneous extrusion of 2 or more polymers of the same or different type, through a single extrusion nozzle, resulting in an extrusion product which, when viewed in section, is in the form of two or more distinct concentric layers. Figure 14 shows a schematic view in section of the co-extruder for preparing the subcutaneous implants of the present invention, where "skin flow" indicates the flow of PLGA used for preparing the film coating (ii) of the subcutaneous implants of the present invention, while "core flow" indicates the flow of the mixture consisting of the active principle dispersed in the PLGA which constitutes the core (i).

In particular the said co-extrusion process comprises the following stages:
a') mixing the active principle with PLGA,
b') possibly granulating the mixture originating from (a') in the minimum solvent quantity, and drying the granules obtained,
c') co-extruding the mixture originating from (a') or from (b') to form the core (i) together with the PLGA optionally in admixture with excipients and/or the active ingredient of the core (i) for preparing the coating in film form(ii).

The coating (ii) in film form presents a thickness of preferably between 5 and 250 µm and more preferably between 10 and 100 µm. Some examples of the preparation of the subcutaneous implants of the present invention are reported by way of illustration in addition to the in-vitro release profiles ensuing thereof.

### EXAMPLE 1 - Preparation of subcutaneous implants containing Avorelin

Subcutaneous implants containing 23.5% mass/mass Avorelin and 76.5% mass/mass PLGA (molar ratio 72/28 - Average molecular weight 115,000 Da) are prepared as described in WO00/33809 and passed for 1 second into a solution of PLGA (molar ratio lactic acid / glycolic acid: 74/26 - Average molecular weight 115,000 Da) in methylene chloride at 173.5 g/l. This is followed by drying the implants treated with said solution in a stream of air. Finally, implants are sterilised by Gamma irradiation at 25 KGy.

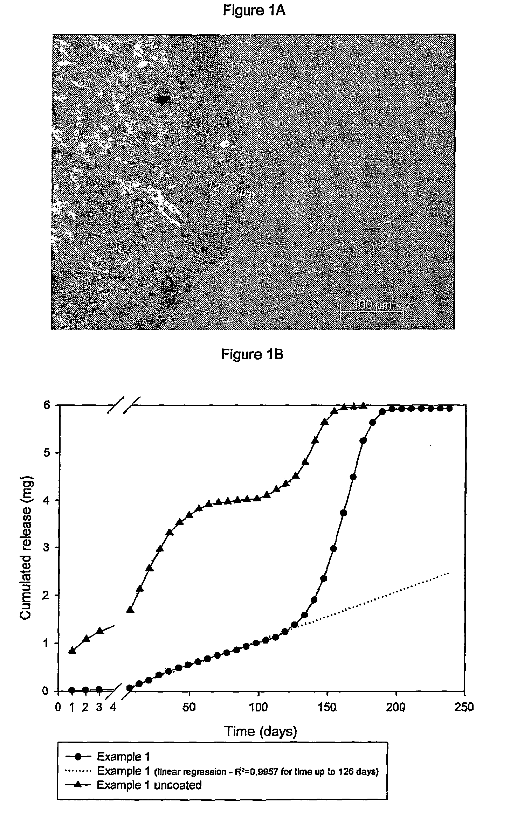

Figure 1A shows an enlarged (150X) cross-section image taken at the above optical microscope of one of the aforesaid coated implats . The coating thickness is about 12 µm in the photographed portion.

Figure 1B shows the in-vitro release profile of the active principle from this type of implant compared with the same uncoated,subcutaneous implant, showing that immediate dissolution of a large amount of the active principle occurred for the uncoated implant (around 0.8 mg on day 1), in contrast to that resulting with the coated subcutaneous implant. In the latter case a linear release (R², i.e. the linearity index calculated according to the minimum square method = 0.9957) occurred over the first 4 months.

### EXAMPLE 2 - Preparation of subcutaneous implants containing Sodium Etidronate

Subcutaneous implants containing 25% mass/mass Sodium Etidronate (water content less than 3.3% mass/mass, residual methanol content: 0.07%, 99.9% purity on dry basis, particle size < 66 µm), and 75% mass/mass polylactic-glycolic acid (PLGA) (molar ratio 54/46 - inherent viscosity 0.56 dl/g measured at 25°C at c = 0.1 g/dl in chloroform) are vigorously mixed.

The mixture in powder form thus obtained was therefore extruded at 100°C. The extrudate thus obtained with a diameter of 1.5 mm was therefore cut to a length of 18 mm resulting in small cylinders each weighing 40 mg (therefore according to that described in the patent application filed in the name of the Applicant simultaneously to the present application) and subsequently allowed to pass into a solution of PLGA in methylene chloride (molar ratio of lactic acid / glycolic acid: 74/26 - Average molecular weight 115,000 Da) at the concentration of 173.5 g/l for 1 second. The implants treated with this solution are subsequently dried in a stream of air. Finally, implants are sterilised by Gamma irradiation at 25 KGy.

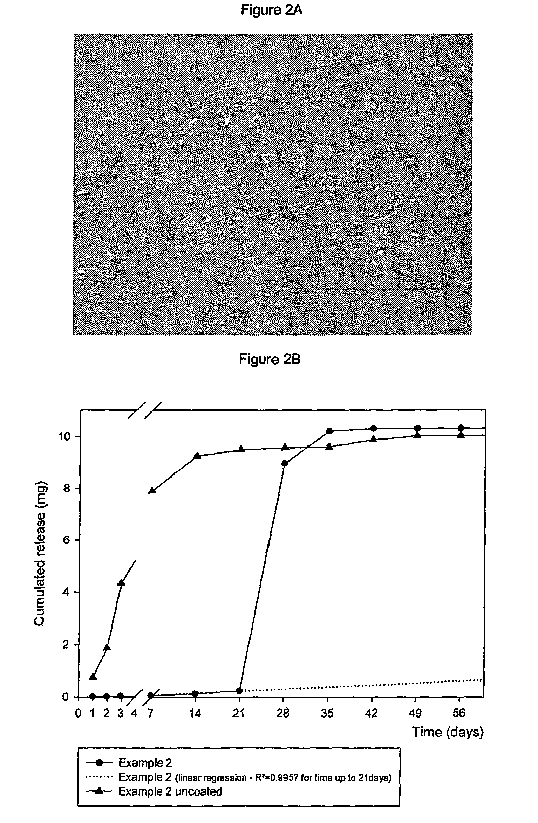

Figure 2A shows the enlarged (300X) cross-section image taken at the above optical microscope of one of the aforesaid deposits. Coating thickness is about 11 µm in the photographed portion.

Figure 2B shows the in-vitro release profile of the active principle of this type of implant compared with the same uncoated subcutaneous implant, highlighting the fact that immediate dissolution of a large amount of the active principle occurred for the uncoated depot (around 2 mg after 2 days), in contrast to that resulting with the coated subcutaneous implant. In the latter case a linear release (R², i.e. the linearity index calculated according to the minimum square method = 0.9957) occurred over the first 3 weeks.

### EXAMPLE 3 - Preparation of subcutaneous implants containing Triptorelin

Subcutaneous implants containing 46% mass/mass of Triptorelin and 54% mass/mass PLGA (molar ratio 72/28 - Average molecular weight 115,000 Da) are prepared as described in WO00/33809 and passed into a solution of PLGA in methylene chloride for 1 second (molar ratio of lactic acid / glycolic acid 74/26 - Average molecular weight: 115,000 Da) at the concentration of 173.5 g/l. The implants treated with this solution are subsequently dried in a stream of air. Finally, implants are sterilised by Gamma irradiation at 25 KGy.

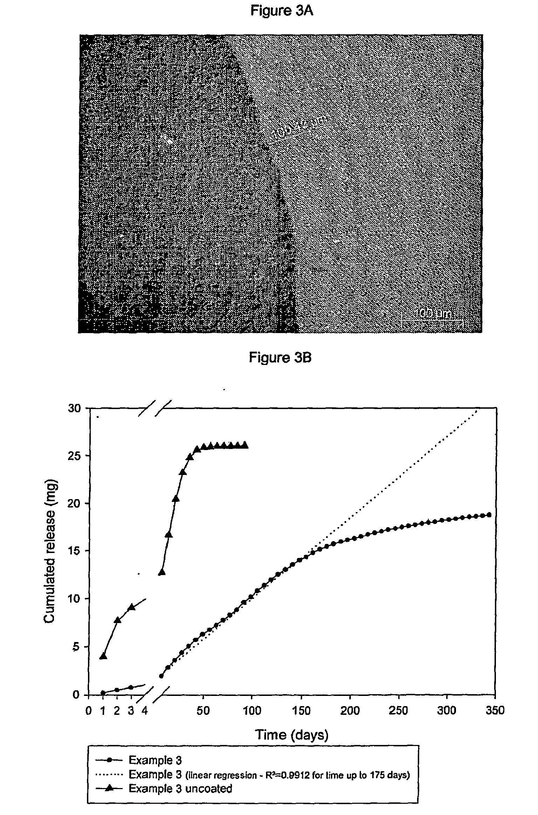

Figure 3A shows the enlarged (150X) cross-section image taken at the above optical microscope of one of the aforesaid coated subcutaneous implants, from which it is noted that the coating thickness is about 100µm.

Figure 3B shows the in-vitro release profile of the active principle of this type of implant compared with the same uncoated subcutaneous implant, highlighting the fact that the immediate dissolution of the active principle is notably reduced in contrast to that resulting with the uncoated subcutaneous implant. In the former case a fairly linear release is obtained (R², i.e. the linearity index, calculated according to the minimum square method = 0.9918 over the first 6 months) with a duration of release of 11 months.

In particular this graph demonstrates that with this type of coated implant the release duration can be considerably prolonged.

### EXAMPLE 4 - Preparation of subcutaneous implants containing Avorelin by coextrusion

Avorelin acetate (50% mass/mass of the total weight of the core) was thoroughly mixed with PLGA (50% mass/mass of the total weight of the core) having the following characteristics:
inherent viscosity 0.19 dl/g measured at 25°C in chloroform (c = 0.1 g/dl),
Lactide / Glycolide Molar ratio: 51/49,
Hydrophilic termination of the chain equivalent to 1 mg of KOH per gram.

The powder mixture was then extruded at 80°C forming the core while the coating was simultaneously formed by coextrusion using the same type of PLGA. During the process, the coextrusion conditions (i.e. amount of material forming coating with respect to the amount of material forming the core passing through the coextrusion die at the same moment) were tuned to obtain 3 different coating thickness (50 µm, 120 µm and 140 µm). The extruded substance obtained (1.6 mm diameter) was then cut at a length of 18 mm, giving rise to 45 mg of a cylindrical coated implants, containing 15, 13 or 11 mg of active principle according to the coating thickness. Finally, implants are sterilised by Gamma irradiation at 25 KGy.

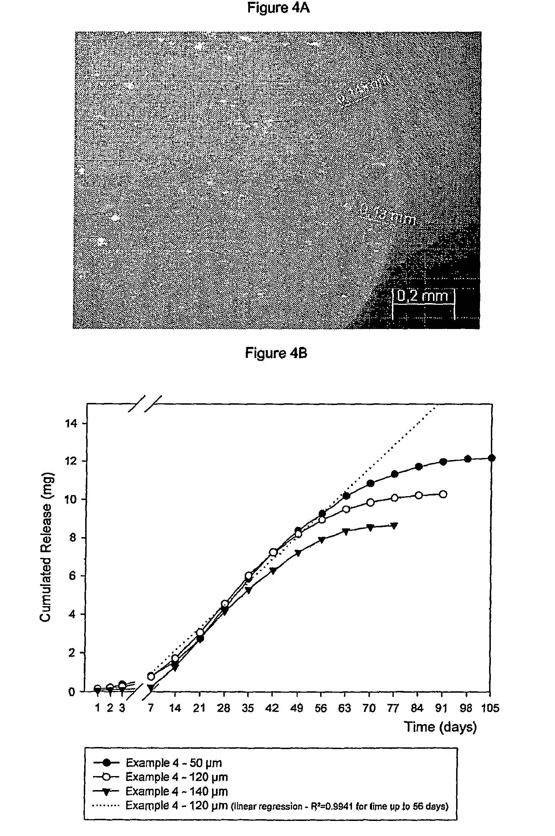

Figure 4A shows an enlarged (75X) cross-section image taken at the above optical microscope of one of the aforesaid coated implants (coating thickness 140 µm).

Figure 4B shows the in vitro release profile of the active principle from the aforesaid cylindrical coated implants.

### EXAMPLE 5 - Preparation of subcutaneous implants containing Avorelin by coextrusion

Avorelin acetate (50% mass/mass of the total weight of the core) was thoroughly mixed with PLGA (50% mass/mass of the total weight of the core) having the following characteristics:
inherent viscosity 0.19 dl/g measured at 25°C in chloroform (c = 0.1 g/dl),
Lactide / Glycolide Molar ratio: 51 / 49,
Hydrophilic termination of the chain equivalent to 1 mg of KOH per gram.

The powder mixture was then extruded at 90°C forming the core while a coating was simultaneously formed by coextrusion using a PLGA having the following characteristics:
inherent viscosity 0.56 dl/g measured at 25°C in chloroform (c= 0.1 g/dl),
Lactide / Glycolide Molar ratio: 56 / 44,
Tg: 39.6°C.

During the process, the coextrusion conditions were tuned to obtain 3 different coating thickness (50 µm, 120 µm and 180 µm). The extruded substance obtained (1.7 mm diameter) was then cut at a length of 18 mm, giving rise to 50 mg of a cylindrical implants, containing 22, 20 or 17 mg of active principle according to the coating thickness. Finally, implants are sterilised by Gamma irradiation at 25 KGy.

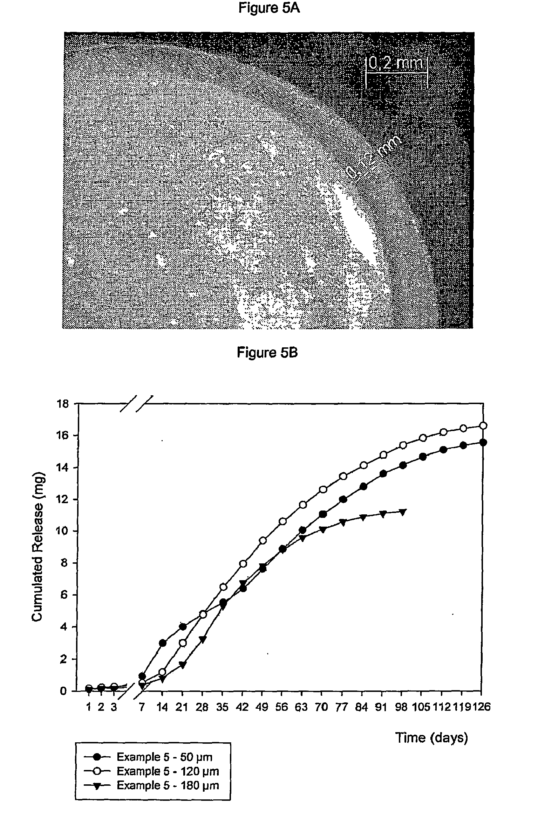

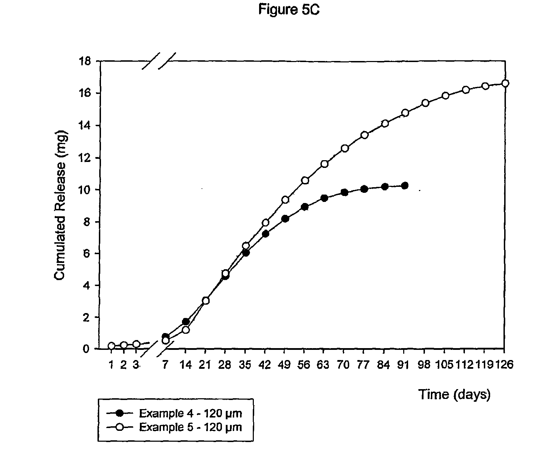

Figure 5A shows an enlarged (75X) cross-section image taken at the above optical microscope of one of the aforesaid subcutaneous implants (coating thickness 120 µm).

Figure 5B shows the in vitro release profile of the active principle from the aforesaid cylindrical coated subcutaneous implants .

Figure 5C shows the in vitro release profile of the active principle from the aforesaid cylindrical subcutaneous implants compared with the corresponding release profile obtained with the Example 4 implants . Example 5 implants differ from the subcutaneous implants disclosed in Example 4 for the molecular weight (and consequently the inherent viscosity) of the PLGA present in the coating. It can be observed that, everything else being equal, using a higher molecular weight PLGA in the coating than in the core leads to longer release duration without affecting the overall release pattern. This is a finding of interest as it shows that it is possible to modulate the release profile by modifying PLGA characteristics in the formula.

### EXAMPLE 6 - Preparation of subcutaneous implants containing Avorelin by coextrusion

Avorelin acetate (50% mass/mass of the total weight of the core) was thoroughly mixed with PLGA (50% mass/mass of the total weight of the core) having the following characteristics:
inherent viscosity 0.56 dl/g measured at 25°C in chloroform (c = 0.1 g/dl),
Lactide / Glycolide Molar ratio: 56 /44,
Tg: 39.6°C.

The powder mixture was then extruded at 90°C forming the core while a skin was simultaneously formed by coextrusion using a PLGA having the following characteristics:
inherent viscosity 0.19 dl/g measured at 25°C in chloroform (c = 0.1 g/dl),
Lactide / Glycolide Molar ratio: 51 / 49,
Hydrophilic termination of the chain equivalent to 1 mg of KOH per gram.

During the process, the coextrusion conditions were tuned to obtain 3 different coating thickness (50 µm, 80 µm and 100 µm). The extruded substance obtained (1.5 mm diameter) was then cut at a length of 18 mm, giving rise to 40 mg of a cylindrical implants , containing 19, 17 or 15 mg of active principle according to the skin thickness. Finally, implants are sterilised by Gamma irradiation at 25 KGy.

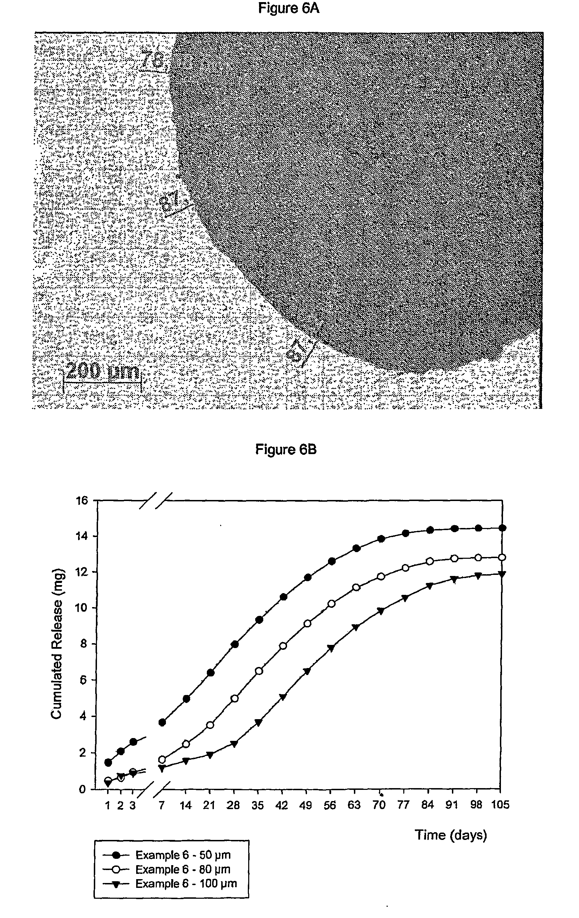

Figure 6A shows an enlarged (75X) cross-section image taken at the above microscope of one of the aforesaid deposits (coating thickness 80 µm).

Figure 6B shows the in vitro release profile of the active principle from the aforesaid cylindrical implants .

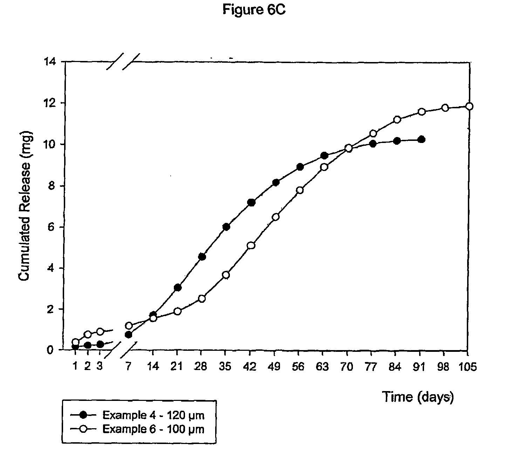

Figure 6C shows the in vitro release profile of the active principle from the aforesaid cylindrical deposits compared with the profile obtained with Example 4 depots. Example 6 depots differ from Example 4 ones for the molecular weight (and consequently the inherent viscosity) of the PLGA present in the core. It can be observed that, everything else being equal, using a higher molecular weight PLGA in the core actually leads to longer release duration. This is a finding of interest as it shows that it is possible to modulate the release profile by modifying PLGA characteristics in the formula.

### EXAMPLE 7 - Preparation of subcutaneous implants containing Avorelin by coextrusion

Avorelin acetate (50% mass/mass of the total weight of the core) was thoroughly mixed with PLGA (50% mass/mass of the total weight of the core) having the following characteristics:
inherent viscosity 0.19 dl/g measured at 25°C in chloroform (c = 0.1 g/dl),
Lactide / Glycolide Molar ratio: 51/49,
Hydrophilic termination of the chain equivalent to 1 mg of KOH per gram.

The powder mixture was then extruded at 80°C forming the core while a coating was simultaneously formed by coextrusion using the same PLGA containing 25% mass/mass of avorelin.

During the process, the coextrusion conditions were tuned to obtain 3 different coating thickness (120 µm, 170 µm and 200 µm). The extruded substance obtained (1.6 mm diameter) was then cut at a length of 18 mm, giving rise to 45 mg of a cylindrical implant, containing 17, 16 or 14 mg of active principle depending on the coating thickness. Finally, implants are sterilised by Gamma irradiation at 25 KGy.

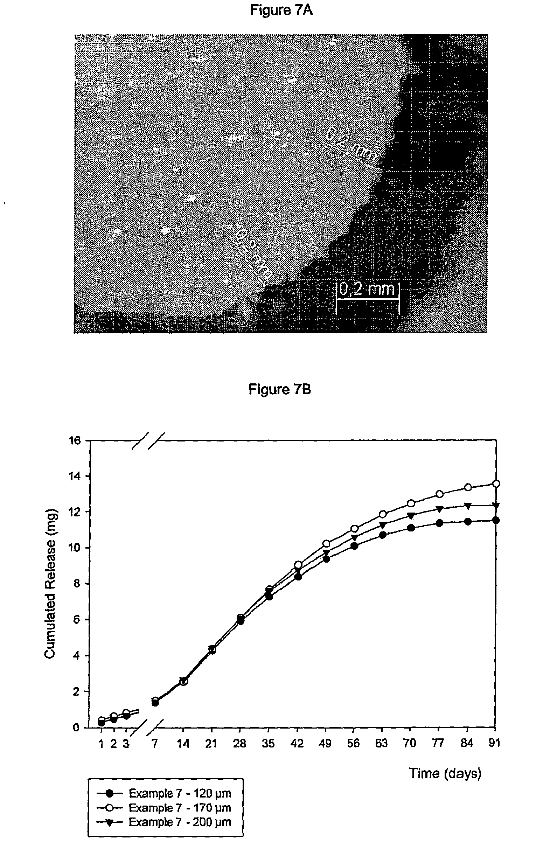

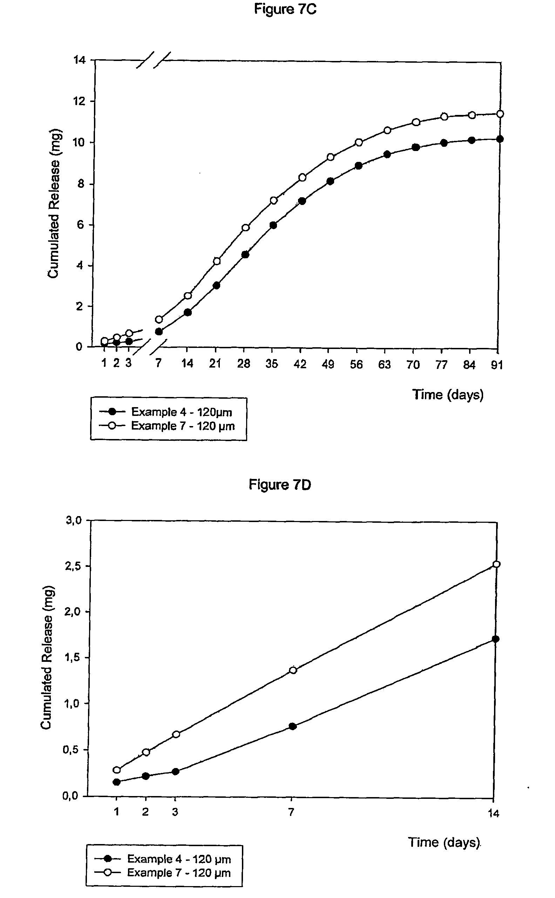

Figure 7A shows the enlarged (75X) cross-section image taken at the above optical microscope of one of the aforesaid subcutaneous implants (coating thickness 200 µm).

Figure 7B shows the in vitro release profile of the active principle from the aforesaid cylindrical implants .

Figure 7C shows the in vitro release profile of the active principle from the aforesaid cylindrical implants compared with the profile obtained with Example 4 implants . Example 7 implants differ from those of Example 4 in that the contains 25% mass/mass of active principle within the PLGA. It can be observed that loading the coating with the active ingredient actually leads to a higher amount released (everything else being equal).

It can also be observed by looking at Figure 7D that the release pattern during the first 2 weeks is much more linear in the case of the implants disclosed in Example 7 than that of the implants disclosed in Example 4. This is a finding of interest as it offers a new possibility to obtain a fairly linear release profile.

### EXAMPLE 8 - Preparation of subcutaneous implants containing Fentanyl citrate

Fentanyl citrate (50% mass/mass based on the total weight of the composition) having the following characteristics:
residual water content: 0.1%,
acetone 1300 ppm,
cyclohexane < 100 ppm,
toluene < 100 ppm,
purity 99.6%,
granulometric distribution 1- 60 µm
was thoroughly mixed with PLGA (50% mass/mass of the total weight of the composition) having the following characteristics:
inherent viscosity 0.56 dl/g measured at 25°C in chloroform (c = 0.1 g/dl),
inherent viscosity 0.50 dl/g measured at 25°C in chloroform (c = 0.5 g/dl),
Tg: 39.6°C.

The powder mixture was then extruded at 105°C. The extruded substance obtained (1.5 mm diameter) was then cut at a length of 18 mm, giving rise to 40 mg of a cylindrical implant, containing 20.7 mg of active principle equal to 51.7% mass/mass (therefore according to that described in the patent application filed in the name of the Applicant simultaneously to the present application). Finally, implants are sterilised by Gamma irradiation at 25 KGy.

### EXAMPLE 9 - Preparation of subcutaneous implants containing Fentanyl citrate by coextrusion

Fentanyl citrate (55% mass/mass of the total weight of the core) having the same characteristics as the one described in the Example 8 was thoroughly mixed with PLGA (45% mass/mass of the total weight of the core) having the following characteristics:
inherent viscosity 0.56 dl/g measured at 25°C in chloroform (c = 0.1 g/dl),
Lactide / Glycolide Molar ratio: 56 /44,
Tg: 39.6°C.

The powder mixture was then extruded at 95°C forming the core while a coating was simultaneously formed by coextrusion using a PLGA having the following characteristics:
inherent viscosity 0.19 dl/g measured at 25°C in chloroform (c = 0.1 g/dl),
Lactide / Glycolide Molar ratio: 51 / 49,
Hydrophilic termination of the chain equivalent to 1 mg of KOH per gram.

During the process, the coextrusion conditions were tuned to obtain 2 different coating thickness (50 and 100 µm). The extruded substance obtained (1.6 mm diameter) was then cut at a length of 18 mm, giving rise to 45 mg of a cylindrical deposit, containing 21 or 17 mg of active principle according to the skin thickness. Finally, implants are sterilised by Gamma irradiation at 25 KGy.

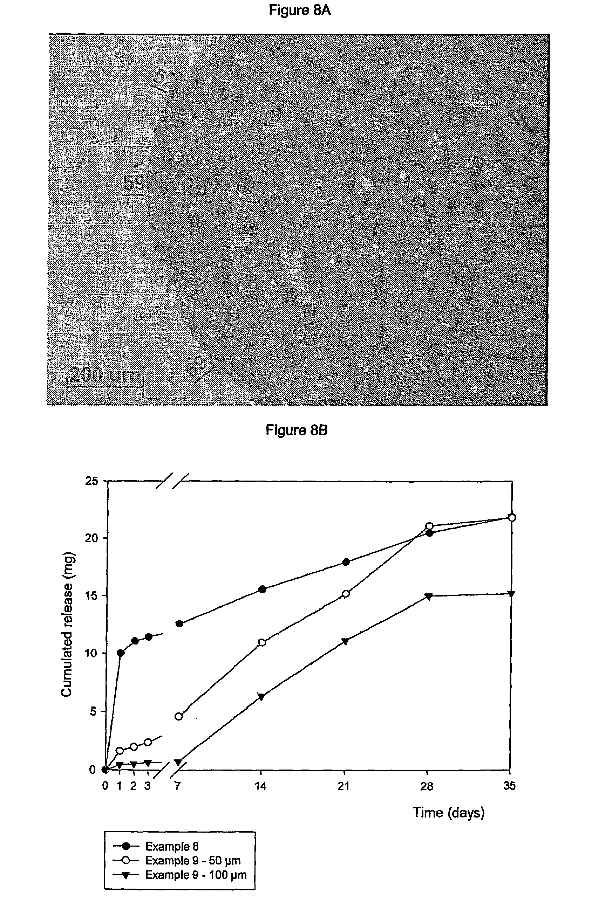

Figure 8A shows an enlarged (75X) cross-section image taken at the above optical microscope of one of the aforesaid implants (thickness coating 50 µm).

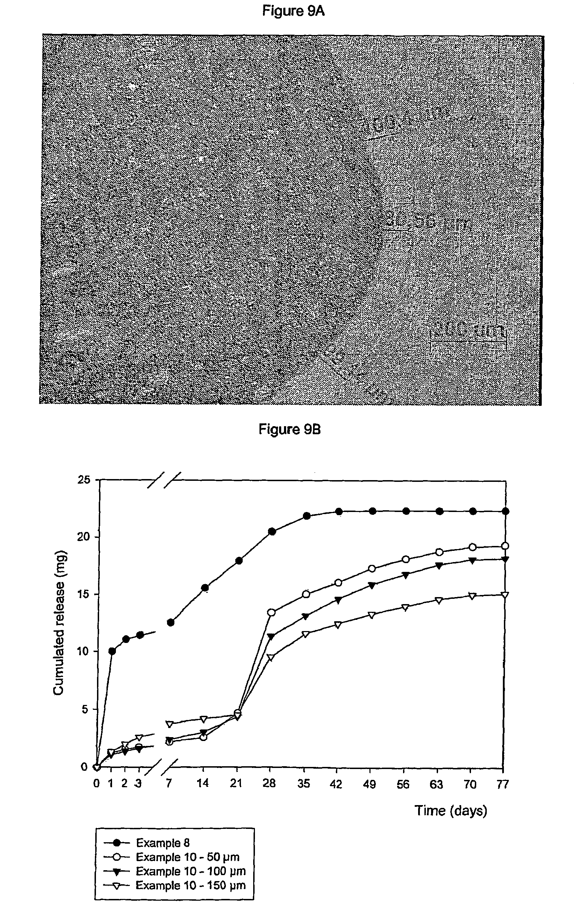

Figure 8B shows the in vitro release profile of the active principle from the aforesaid cylindrical coated implants compared with that obtained with implants disclosed in Example 8.

### EXAMPLE 10 - Preparation of subcutaneous implants containing Fentanyl citrate by coextrusion

Fentanyl citrate (55% mass/mass of the total weight of the core) having the same characteristics as the one described in the Example 8 was thoroughly mixed with PLGA (45% mass/mass of the total weight of the core) having the following characteristics:
inherent viscosity 1.05 dl/g measured at 25°C in chloroform (c = 0.1 g/dl),
Lactide / Glycolide Molar ratio: 74 / 26,
Tg: 49.1 °C.

The powder mixture was then extruded at 105°C forming the core while a coating was simultaneously formed by coextrusion using a PLGA having the following characteristics:
inherent viscosity 0.56 dl/g measured at 25°C in chloroform (c = 0.1 g/dl),
Lactide / Glycolide Molar ratio: 56 / 44,
Tg: 39.6°C.

During the process, the coextrusion conditions were tuned to obtain 3 different coating thickness (50 µm, 100 µm and 150 µm). The extruded substance obtained (1.6 mm diameter) was then cut at a length of 18 mm, giving rise to 45 mg of a cylindrical deposit, containing 22, 19 or 15 mg of active principle according to the coating thickness. Finally, implants are sterilised by Gamma irradiation at 25 KGy.

Figure 9A shows an enlarged (75X) cross-section image taken at the above optical microscope of one of the aforesaid implants (coating thickness 100 µm).

Figure 9B shows the in vitro release profile of the active principle from the aforesaid cylindrical implants.

### EXAMPLE 11 - Preparation of subcutaneous implants containing Fentanyl citrate by coextrusion

Fentanyl citrate (55% mass/mass of the total weight of the core) having the same characteristics that the one described in the Example 8 was thoroughly mixed with PLGA (45% mass/mass of the total weight of the core) having the following characteristics:
inherent viscosity 0.56 dl/g measured at 25°C in chloroform (c = 0.1 g/dl),
Lactide / Glycolide Molar ratio: 56 / 44,
Tg: 39.6°C.

The powder mixture was then extruded at 95°C forming the core while a coating was simultaneously formed by coextrusion using a PLGA having the following characteristics:
inherent viscosity 0.56 dl/g measured at 25°C in chloroform (c = 0.1 g/dl),
Lactide / Glycolide Molar ratio: 56 / 44,
Tg: 39.6°C.

During the process, the coextrusion conditions were tuned to obtain 3 different coating thickness (100 µm, 150 µm and 200 µm). The extruded substance obtained (1.6 mm diameter) was then cut at a length of 18 mm, giving rise to 45 mg of a cylindrical implant, containing 18, 16 or 14 mg of active principle according to the coating thickness. Finally, implants are sterilised by Gamma irradiation at 25 KGy.

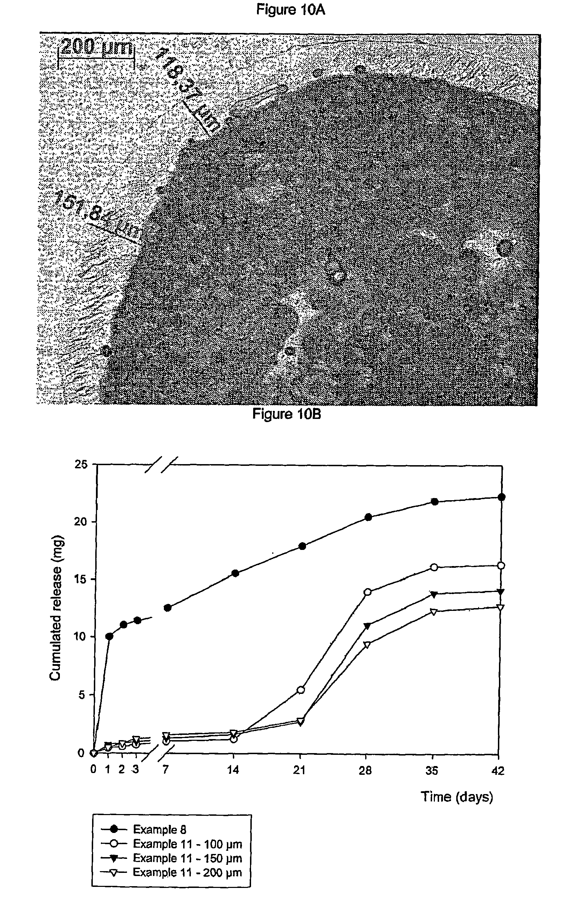

Figure 10A shows an enlarged (75X) cross-section image taken at the above optical microscope of one of the aforesaid implants (coating thickness 150 µm).

Figure 10B shows the in vitro release profile of the active principle from the aforesaid cylindrical subcutaneous implants .

### EXAMPLE 12 - Preparation of subcutaneous implants containing Fentanyl citrate by coextrusion

Fentanyl citrate (55% mass/mass of the total weight of the core) having the same characteristics as the one described in the Example 8 was thoroughly mixed with PLGA (45% mass/mass of the total weight of the core) having the following characteristics:
inherent viscosity 1.05 dl/g measured at 25°C in chloroform (c = 0.1 g/dl),
Lactide / Glycolide Molar ratio: 74 / 26,
Tg: 49.1 °C.

The powder mixture was then extruded at 105°C forming the core while a coating was simultaneously formed by coextrusion using a mix of 20% mass/mass of Polyvinylpyrrolidone and 80% mass/mass of PLGA having the following characteristics:
inherent viscosity 0.56 dl/g measured at 25°C in chloroform (c = 0.1 g/dl),
Lactide / Glycolide Molar ratio: 56 / 44,
Tg: 39.6°C.

During the process, the coextrusion conditions were tuned to obtain a coating thickness of 150 µm. The extruded substance obtained (1.6 mm diameter) was then cut at a length of 18 mm, giving rise to 45 mg of a cylindrical deposit, containing 17 mg of active principle. Finally, implants are sterilised by Gamma irradiation at 25 KGy.

### EXAMPLE 13 - Preparation of subcutaneous implants containing Fentanyl citrate by coextrusion

Fentanyl citrate (55% mass/mass of the total weight of the core) having the same characteristics as the one described in the Example 8 was thoroughly mixed with PLGA (45% mass/mass of the total weight of the core) having the following characteristics:
inherent viscosity 1.05 dl/g measured at 25°C in chloroform (c = 0.1 g/dl),
Lactide / Glycolide Molar ratio: 74 / 26,
Tg: 49.1 °C.

The powder mixture was then extruded at 105°C forming the core while a coating was simultaneously formed by coextrusion using a mix of 20% mass/mass of D-mannitol and 80% mass/mass of PLGA having the following characteristics:
inherent viscosity 0.56 dl/g measured at 25°C in chloroform (c = 0.1 g/dl),
Lactide / Glycolide Molar ratio: 56 / 44,
Tg: 39.6°C.

During the process, the coextrusion conditions were tuned to obtain a coating thickness of 150 µm. The extruded substance obtained (1.6 mm diameter) was then cut at a length of 18 mm, giving rise to 45 mg of a cylindrical deposit, containing 17 mg of active principle. Finally, implants are sterilised by Gamma irradiation at 25 KGy.

Figure 11 shows the in vitro release profile of the active principle from the aforesaid cylindrical implants compared with those obtained through Examples 12 and 10 (coating thickness 150 µm).

### EXAMPLE 14 - Preparation of subcutaneous implants containing Medroxyprogesterone acetate

Medroxyprogesterone acetate of pharmacopoeia specification (55% mass/mass of the total weight) and polylactic-glycolic acid (45% mass/mass of the total weight) having the following characteristics:
DL lactide / glycolide molar ratio 74/26,
inherent viscosity 1.05 dl/g measured at 25°C in chloroform (c = 0.1 g/dl),
Tg: 49.1°C
were thoroughly dry-mixed. The mixture thus obtained was extruded at 120°C. The extruded product obtained, having a diameter of 1.8 mm, was then cut at a length of 18 mm to obtain subcutaneous implants each weighing 60 mg and containing 30 mg of active principle.

### EXAMPLE 15 - Preparation of subcutaneous implants containing Medroxyprogesterone acetate by coextrusion

Medroxyprogesterone acetate (55% mass/mass of the total weight of the core) of pharmacopoeia specification was thoroughly mixed with PLGA (45% mass/mass of the total weight of the core) having the following characteristics:
inherent viscosity 1.05 dl/g measured at 25°C in chloroform (c = 0.1 g/dl),
Lactide / Glycolide Molar ratio: 74 / 26,
Tg: 49.1 °C.

The powder mixture was then extruded at 105°C forming the core while a coating was simultaneously formed by coextrusion using a PLGA having the following characteristics:
inherent viscosity 1.05 dl/g measured at 25°C in chloroform (c = 0.1 g/dl),
Lactide / Glycolide Molar ratio: 74 /26,
Tg: 49.1 °C.

During the process, the coextrusion conditions were tuned in order to obtain a coating thickness of 150 µm. The extruded substance obtained (1.9 mm diameter) was then cut at a length of 18 mm, giving rise to 60 mg of a cylindrical deposit, containing 20 mg of active principle. Finally, implants are sterilised by Gamma irradiation at 25 KGy.

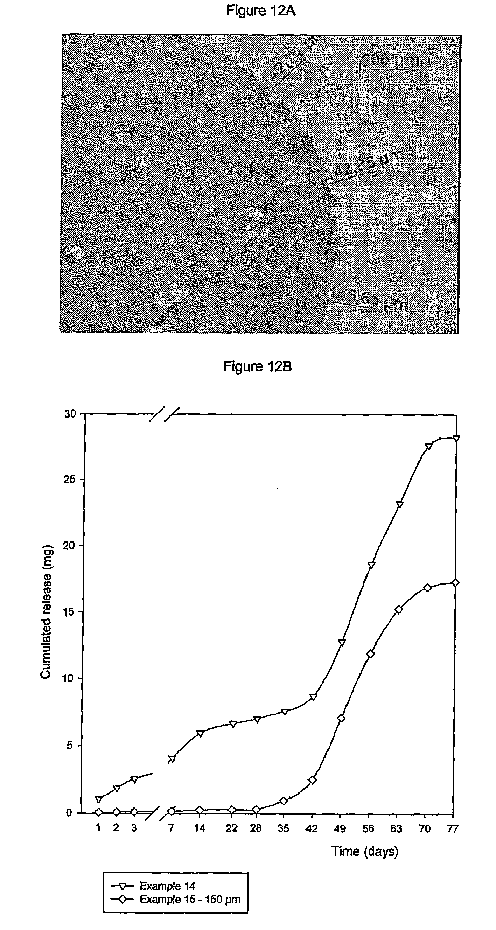

Figure 12A shows an enlarged (75X) cross-section image taken at the above optical microscope of one of the aforesaid implants.

Figure 12B shows the in vitro release profile of the active principle from the aforesaid cylindrical implants compared with the corresponding release profile obtained with the uncoated subcutaneous implant disclosed in Example 14.

### EXAMPLE 16 - Preparation of subcutaneous implants containing Medroxyprogesterone acetate by coextrusion

Medroxyprogesterone acetate (55% mass/mass of the total weight of the core) of pharmacopoeia specification was thoroughly mixed with PLGA (45% mass/mass of the total weight of the core) having the following characteristics:
inherent viscosity 0.56 dl/g measured at 25°C in chloroform (c = 0.1 g/dl),
Lactide / Glycolide Molar ratio: 56 / 44,
Tg: 39.6°C.

The powder mixture was then extruded at 105°C forming the core while a coating was simultaneously formed by coextrusion using a PLGA having the following characteristics:
inherent viscosity 0.19 dl/g measured at 25°C in chloroform (c = 0.1 g/dl),
Lactide / Glycolide Molar ratio: 51 / 49,
Hydrophilic termination of the chain equivalent to 1 mg of KOH per gram.

During the process, the coextrusion conditions were tuned to obtain a coating thickness of 150 µm. The extruded substance obtained (1.9 mm diameter) was then cut at a length of 18 mm, giving rise to 60 mg of a cylindrical implant containing 20 mg of active principle. Finally, implants are sterilised by Gamma irradiation at 25 KGy.

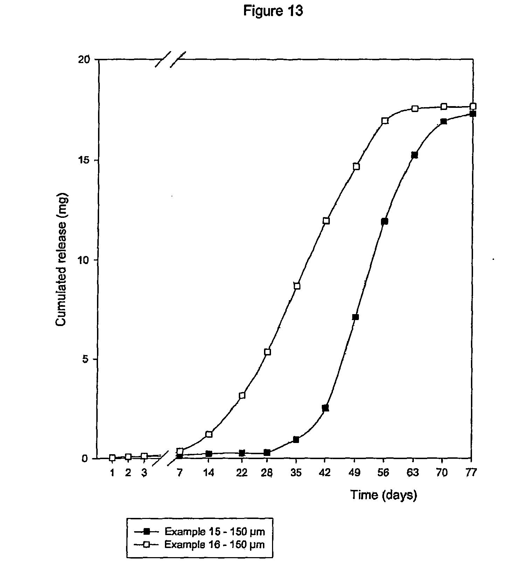

Figure 13 shows the in vitro release profile of the active principle from the aforesaid cylindrical implants compared with the one obtained with implants disclosed in Example 15.

## Claims

1. Subcutaneous implants comprising:
- a core (i) comprising at least one active principle dispersed in a polymeric matrix essentially consisting of PLGA obtained by extrusion,
- a coating (ii) in film form comprising as the main component PLGA;

2. Subcutaneous implant as claimed in claim 1, wherein the active principle contained in the core (i) is chosen from the class consisting of: a peptide, an active principle able to increase bone density, an analgesic-narcotic, a steroid hormone for hormonal treatments during menopause or for contraception.

3. Subcutaneous implant as claimed in claim 2, **characterised in that** when the core (i) contains a peptide the particles of said active principle present extremely heterogeneous dimensions which vary from 1 micron to 63 microns.

4. Subcutaneous implants as claimed in any one of claims 1-3, **characterised in that** the PLGA used in the core (i) presents a molecular weight between 50,000 and 150,000 and a molar ratio of lactic acid to glycolic acid monomers between 50:50 and 95:5.

5. Subcutaneous implants as claimed in anyone of claims 1-4, wherein the coating (ii) contains PLGA in amounts ranging from 75 to 99,999% and the remaining to 100 essentially consisting of excipients and/or of the same active ingredient used in the core (i).

6. The subcutaneous implants according to claim 5, wherein the coating (ii) essentially consists of PLGA.

7. The subcutaneous implants according to claim 5, wherein the coating (ii) consists of a mixture of 80%PLGA and the remaining to 100% of at least one hydrophilic excipient.

8. The subcutaneous implants according to claim 7, wherein said hydrophilic excipient is selected from the group consisting of polyvinyl pyrrolidone, D-mannitol and mixtures thereof.

9. The subcutaneous implants according to claim 5, wherein the coating (ii) consists of a mixture of 75% PLGA and the remaining to 100% of the same active ingredient contained in the core (i).

10. Subcutaneous implant as claimed in any one of claims 1-9, **characterised in that** said coating in film form (ii) consists of PLGA with a molecular weight between 50,000 and 150,000 and a molar ratio of lactic acid to glycolic acid monomers between 50:50 and 95:5.

11. Subcutaneous implant as claimed in claim 10, wherein said PLGA presents an average molecular weight between 100,000 and 150,000 and said molar ratio is comprised between 50/50 and 75/25.

12. Subcutaneous implant as claimed in any one of claims 1-11, **characterised in that** the coating (ii) presents a thickness between 5 and 250 µm.

13. Subcutaneous implant as claimed in claim 12, wherein said thickness is comprised between 10 and 100 µm.

14. Process for preparing the subcutaneous implants as claimed in anyone of claims 1-13, comprising the following stages:
a) preparing the core (i) containing the active principle by extrusion,
b) passing the core (i) into a solution of PLGA in a suitable solvent chosen from apolar and aprotic polar solvent such that said cores remain in contact with said solution for a period between 1 and 5 seconds,
c) drying said cores originating from stage (b).

15. Process as claimed in claim 14, wherein the apolar solvent is a chlorinated solvent.

16. Process as claimed in claim 15, **characterised in that** said solvent is methylene chloride.

17. Process as claimed in claim 14, wherein said aprotic polar solvent is chosen from acetonitrile, ethyl acetate, tetrahydrofuran.

18. Process as claimed in any one of claims 14-17, wherein the PLGA concentration in the solution used in stage (a) is comprised between 70 and 300 g/l.

19. Process as claimed in claim 18, wherein said concentration is comprised between 100 and 200 g/l.

20. Process as claimed in any one of claims 14-19, **characterised in that** said contact time is 1 second.

21. Process for preparing the subcutaneous implant in according to any one of claims 1-13 comprising the following stages:
a') mixing the active principle with PLGA,
b') possibly granulating the mixture originating from (a') in the minimum solvent quantity, and drying the granules obtained,
c') co-extruding the mixture originating from (a') or from (b') together with the PLGA used for preparing the coating in film form (ii).

## Patentansprüche

1. Subkutane Implantate, umfassend:
- einen Kern (i) umfassend mindestens einen Wirkstoff, dispergiert in einer Polymermatrix, im wesentlichen bestehend aus PLGA, erhalten durch Extrusion,
- eine Beschichtung (ii) in Filmform, umfassend PLGA als Hauptkomponente.

2. Subkutanes Implantat gemäß Anspruch 1, wobei der Wirkstoff, der in dem Kern (i) enthalten ist, aus der Klasse ausgewählt wird bestehend aus: einem Peptid, einem Wirkstoff, der die Knochendichte erhöhen kann, einem Analgetikum-Narkotikum, einem Steroidhormon für Hormonbehandlungen während der Menopause oder zur Empfängnisverhütung.

3. Subkutanes Implantat gemäß Anspruch 2, **dadurch gekennzeichnet, daß** wenn der Kern (i) ein Peptid enthält, die Teilchen des Wirkstoffs extrem heterogene Dimensionen präsentieren, die von 1 bis 63 µm variieren.

4. Subkutane Implantate gemäß einem der Ansprüche 1 bis 3, **dadurch gekennzeichnet, daß** das im Kern (i) verwendete PLGA ein Molekulargewicht zwischen 50.000 und 150.000 und ein molares Verhältnis von Milchsäure zu Glycolsäuremonomeren zwischen 50:50 und 95:5 präsentiert.

5. Subkutane Implantate gemäß einem der Ansprüche 1 bis 4, wobei die Beschichtung (ii) PLGA in Mengen im Bereich von 75 bis 99,999 % enthält und das auf 100 Verbleibende im wesentlichen aus Exzipienzien und/oder demselben Wirkstoff, der im Kern (i) verwendet wird, besteht.

6. Subkutane Implantate gemäß Anspruch 5, wobei die Beschichtung (ii) im wesentlichen aus PLGA besteht.

7. Subkutane Implantate gemäß Anspruch 5, wobei die Beschichtung (ii) aus einer Mischung aus 80 % PLGA besteht und das Verbleibende auf 100 % aus mindestens einem hydrophilen Exzipienz.

8. Subkutane Implantate gemäß Anspruch 7, wobei das hydrophile Exzipienz ausgewählt wird aus der Gruppe bestehend aus Polyvinylpyrrolidon, D-Mannit und Mischungen daraus.

9. Subkutane Implantate gemäß Anspruch 5, wobei die Beschichtung (ii) aus einer Mischung aus 75 % PLGA besteht und das Verbleibende auf 100 % aus demselben Wirkstoff, der auch im Kern (i) enthalten ist.

10. Subkutanes Implantat gemäß einem der Ansprüche 1 bis 9, **dadurch gekennzeichnet, daß** die Beschichtung in Filmform (ii) aus PLGA mit einem Molekulargewicht zwischen 50.000 und 150.000 und einem molaren Verhältnis von Milchsäure zu Glycolsäuremonomeren zwischen 50:50 und 95:5 besteht.

11. Subkutanes Implantat gemäß Anspruch 10, wobei das PLGA ein durchschnittliches Molekulargewicht zwischen 100.000 und 150.000 präsentiert und wobei das molare Verhältnis zwischen 50/50 und 75/25 liegt.

12. Subkutanes Implantat gemäß einem der Ansprüche 1 bis 11, **dadurch gekennzeichnet, daß** die Beschichtung (ii) eine Dicke zwischen 5 und 250 µm präsentiert.

13. Subkutanes Implantat gemäß Anspruch 12, wobei die Dicke zwischen 10 und 100 µm liegt.

14. Verfahren zur Herstellung der subkutanen Implantate gemäß einem der Ansprüche 1 bis 13, umfassend die folgenden Stufen:
a) Herstellung des Kerns (i), enthaltend den Wirkstoff, durch Extrusion,
b) Weiterführen des Kerns (i) in eine Lösung aus PLGA in einem geeigneten Lösungsmittel, ausgewählt aus einem apolaren oder aprotischen polaren Lösungsmittel, so daß die Kerne für eine Zeitspanne zwischen 1 und 5 Sekunden in Kontakt mit der Lösung bleiben,
c) Trocknen der Kerne, die sich aus Stufe (b) ergeben.

15. Verfahren gemäß Anspruch 14, wobei das apolare Lösungsmittel ein chloriertes Lösungsmittel ist.

16. Verfahren gemäß Anspruch 15, **dadurch gekennzeichnet, daß** das Lösungsmittel Methylenchlorid ist.

17. Verfahren gemäß Anspruch 14, wobei das aprotische polare Lösungsmittel ausgewählt ist aus Acetonitril, Ethylacetat, Tetrahydrofuran.

18. Verfahren gemäß einem der Ansprüche 14 bis 17, wobei die PLGA-Konzentration in der in Stufe (a) verwendeten Lösung zwischen 70 und 300 g/l liegt.

19. Verfahren gemäß Anspruch 18, wobei die Konzentration zwischen 100 und 200 g/l liegt.

20. Verfahren gemäß einem der Ansprüche 14 bis 19, **dadurch gekennzeichnet, daß** die Kontaktzeit 1 Sekunde ist.

21. Verfahren zur Herstellung des subkutanen Implantats gemäß einem der Ansprüche 1 bis 13, umfassend die folgenden Stufen:
a') Vermischen des Wirkstoffs mit PLGA,
b') möglicherweise Granulieren der Mischung, die sich aus Stufe (a') ergibt in einer minimalen Lösungsmittelmenge und Trocknen der erhaltenen Körner,
c') Co-Extrusion der Mischung, die sich aus Stufe (a') oder (b') ergibt, mit dem PLGA, das verwendet wird, um die Beschichtung in Filmform (ii) herzustellen.

## Revendications

1. Implants sous-cutanés comprenant:
- un coeur (i) comprenant au moins un principe actif dispersé dans une matrice polymérique constituée essentiellement de PLGA obtenu par extrusion;
- un revêtement (ii) sous forme de film comprenant du PLGA en tant que composant principal.

2. Implant sous-cutané selon la revendication 1, dans lequel le principe actif contenu dans le coeur (i) est choisi dans le groupe comprenant : un peptide, un principe actif capable d'augmenter la densité osseuse, un narcotique-analgésique, un stéroïde hormonal pour les traitements hormonaux au cours de la ménopause ou pour la contraception.

3. Implant sous-cutané selon la revendication 2, **caractérisé en ce que**, lorsque le coeur (i) contient un peptide, les particules dudit principe actif présentent des dimensions extrêmement hétérogènes qui varient entre 1 micron et 63 microns.

4. Implants sous-cutanés selon l'une quelconque des revendications 1 à 3, **caractérisé en ce que** le PLGA utilisé dans le coeur (i) présente une masse moléculaire comprise entre 50000 et 150000, et un rapport molaire entre les monomères d'acide lactique et les monomères d'acide glycolique compris entre 50:50 et 95:5.

5. Implants sous-cutanés selon l'une quelconque des revendications 1 à 4, dans lesquels le revêtement (ii) comprend une quantité de PLGA comprise entre 75 et 99,999%, le pourcentage restant étant essentiellement constitué de d'excipients et/ou du même ingrédient actif que celui utilisé dans le coeur (i).

6. Implants sous-cutanés selon la revendication 5, dans lesquels le revêtement (ii) est essentiellement constitué de PLGA.

7. Implants sous-cutanés selon la revendication 5, dans lesquels le revêtement (ii) est constitué d'un mélange de 80% de PLGA et du pourcentage restant constitué par 100% d'au moins un excipient hydrophile.

8. Implants sous-cutanés selon la revendication 7, dans lesquels ledit excipient hydrophile est choisi dans le groupe constitué par la polyvinyl pyrrolidone, le D-mannitol et des mélanges de ceux-ci.

9. Implants sous-cutanés selon la revendication 5, dans lesquels le revêtement (ii) est constitué par un mélange de 75% de PLGA et du pourcentage restant constitué du même ingrédient actif que celui contenu dans le coeur (i).

10. Implant sous-cutané selon l'une quelconque des revendications 1 à 9, **caractérisé en ce que** ledit revêtement sous forme de film (ii) est constitué par du PLGA de masse moléculaire comprise entre 50000 et 150000 et de rapport molaire entre les monomères d'acide lactique et les monomères d'acide glycolique compris entre 50:50 et 95:5.

11. Implant sous-cutané selon la revendication 10, dans lequel le PLGA présente une masse moléculaire moyenne comprise entre 100000 et 150000 et ledit rapport molaire est compris entre 50/50 et 75/25.

12. Implant sous-cutané selon l'une quelconque des revendications 1 à 11, **caractérisé en ce que** le revêtement (ii) présente une épaisseur comprise entre 5 et 250 µm.

13. Implant sous-cutané selon la revendication 12, dans lequel ladite épaisseur est comprise entre 10 et 100 µm.

14. Procédé de préparation d'implants sous-cutanés selon l'une quelconque des revendications 1 à 13, comprenant les étapes suivantes :
a) préparation du coeur (i) contenant le principe actif par extrusion;
b) passage du coeur (i) dans une solution de PLGA dans un solvant approprié choisi parmi les solvants apolaires et aprotiques polaires de sorte que ledit coeur demeure en contact avec ladite solution pendant une durée comprise entre 1 et 5 secondes;
c) séchage dudit coeur provenant de l'étape b).

15. Procédé selon la revendication 14, dans lequel le solvant apolaire est un solvant chloré.

16. Procédé selon la revendication 15, **caractérisé en ce que** ledit solvant est le chlorure de méthylène.

17. Procédé selon la revendication 14, dans lequel ledit solvant aprotique polaire est choisi parmi l'acétonitrile, l'acétate d'éthyle et le tétrahydrofuranne.

18. Procédé selon l'une quelconque des revendications 14 à 17, dans lequel la concentration de PLGA dans la solution utilisée dans l'étape a) est comprise entre 70 et 300 g/l.

19. Procédé selon la revendication 18, dans lequel ladite concentration est comprise entre 100 et 200 g/l.

20. Procédé selon l'une quelconque des revendications 14 à 19, **caractérisé en ce que** ledit temps de contact est de 1 seconde.

21. Procédé de préparation d'un implant sous-cutané selon l'une quelconque des revendications 1 à 13 comprenant les étapes suivantes:
a') mélange du principe actif avec PLGA,
b') granulation éventuelle du mélange issu de l'étape a') dans une quantité minimale de solvant, et séchage des granules obtenus,
c') co-extrusion du mélange issu de l'étape a') ou de l'étape b') avec le PLGA utilisé pour la préparation du revêtement sous forme de film (ii).
